# FASCICULE DE BREVET EUROPEEN

(11) **EP 0 783 585 B1**
(45) Date de publication et mention de la délivrance du brevet: **28.06.2006**
(21) Numéro de dépôt: 95932050.8
(22) Date de dépôt: 22.09.1995
(51) Int. Cl.: C12N 15/86, C07K 14/075

(54) **ADENOVIRUS RECOMBINANTS DEFECTIFS AVEC UN GENE IVa2 INACTIVE**
DEFEKTIVE REKOMBINANTE ADENOVIREN MIT EINEM INAKTIVIERTEN GEN IV A 2
DEFFECTIVE RECOMBINANT ADENOVIRUSES WITH INACTIVATED IVa2 GENE

(30) Priorité: 27.09.1994 FR 9411511
(43) Date de publication de la demande: 16.07.1997
(73) Titulaire: Aventis Pharma S.A., 92160 Antony (FR)
(72) Inventeur: ORSINI, Cécile, F-75012 Paris (FR); PERRICAUDET, Michel, F-28320 Ecrosnes (FR); VIGNE, Emmanuelle, F-94200 Ivry-sur-Seine (FR); YEH, Patrice, F-75005 Paris (FR)
(74) Mandataire: Schmitz, Jean-Marie
(86) Numéro de dépôt international: PCT/FR1995/001228
(87) Numéro de publication internationale: WO 1996/010088

(56) Documents cités:
- FR-A- 2 707 664
- PROCEEDINGS OF THE NATIONAL ACADEMY OF SCIENCES OF USA., vol. 81, no. 20, Octobre 1984 WASHINGTON US, pages 6290-6294, NATARAJAN, V. ET AL. 'Proximal and distal domain that control in vitro transcription of the adenovirus IVa2 gene'

## Description

La présente invention concerne de nouveaux vecteurs viraux, leur préparation et leur utilisation en thérapie génique. Elle concerne également les compositions pharmaceutiques contenant lesdits vecteurs viraux. Plus particulièrement, la présente invention concerne des adénovirus recombinants comme vecteurs pour la thérapie génique.

La thérapie génique consiste à corriger une déficience ou une anomalie (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de ΔEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

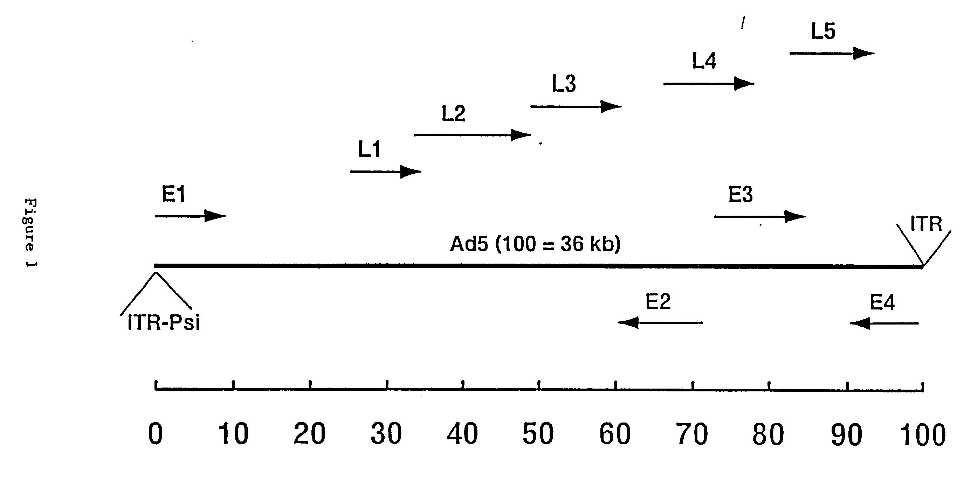

Parmi ces virus, les adénovirus présentent certaines propriétés intéressantes pour une utilisation en thérapie génique. Notamment, ils ont un spectre d'hôte assez large, sont capables d'infecter des cellules quiescentes, ne s'intègrent pas au génome de la cellule infectée, et n'ont pas été associés à ce jour à des pathologies importantes chez l'homme. Les adénovirus sont des virus à ADN double brin linéaire d'une taille de 36 kb environ. Leur génome comprend notamment une séquence inversée répétée (ITR) à leur extrémité, une séquence d'encapsidation, des gènes précoces et des gènes tardifs (Cf figure 1). Les principaux gènes précoces sont les gènes E1 (E1a et E1b), E2, E3 et E4. Les principaux gènes tardifs sont les gènes L1 à L5.

Compte tenu des propriétés des adénovirus mentionnées ci-dessus, ceux-ci ont déjà été utilisés pour le transfert de gènes in vivo. A cet effet, différents vecteurs dérivés des adénovirus ont été préparés, incorporant différents gènes (β-gal, OTC, a-lAT, cytokines, etc). Dans chacune de ces constructions, l'adénovirus a été modifié de manière à le rendre incapable de réplication dans la cellule infectée. Ainsi, les constructions décrites dans l'art antérieur sont des adénovirus délétés des régions E1 (E1a et/ou E1b) et éventuellement E3 au niveau desquelles sont insérées les séquences d'ADN hétérologue (Levrero et al., Gene 101 (1991) 195; Gosh-Choudhury et al., Gene 50 (1986) 161). Néanmoins, les vecteurs décrits dans fart antérieur présentent de nombreux inconvénients qui limitent leur exploitation en thérapie génique. En particulier, tous ces vecteurs comportent de nombreux gènes viraux dont l'expression in vivo n'est pas souhaitable dans le cadre d'une thérapie génique. De plus, ces vecteurs ne permettent pas l'incorporation de fragments d'ADN de très grande taille, qui peuvent être nécessaires pour certaines applications.

La présente invention permet de remédier à ces inconvénients. La présente invention décrit en effet de nouveaux adénovirus recombinants utilisables en thérapie génique, notamment pour le transfert et l'expression de gènes in vivo. En particulier, les adénovirus de l'invention permettent un transfert efficace et une expression durable in vivo d'un gène d'intérêt, tout en limitant les risques de production de protéines virales, de transmission du virus, de pathogénicité, etc.

La présente invention réside plus particulièrement dans la construction d'adénovirus recombinants dans lesquels au moins un gène viral particulier, désigné IVa2, a été rendu non fonctionnel. Le gène IVa2 est un petit gène situé dans la partie gauche du génome de l'adénovirus. Il se chevauche en partie avec l'extrémité du gène E2B notamment, ce qui rend délicate sa manipulation. Le gène IVa2 semble jouer un rôle d'activateur de la transcription des gènes tardifs dans le cycle réplicatif de l'adénovirus. Sa présence permet ou favorise donc l'expression des protéines du virus, nécessaires à la formation des particules virales.

La demanderesse a maintenant montré qu'il est possible d'inactiver ce gène dans le génome de l'adénovirus. La demanderesse a par ailleurs montré que cette inactivation n'affectait pas les propriétés de l'adénovirus comme vecteur de thérapie génique, à savoir son haut pouvoir d'infection des cellules, notamment humaines, et sa capacité à transferer efficacement une gène d'intérêt dans lesdites cellules. De plus, par rapport aux vecteurs de l'art antérieur, les vecteurs décrits dans la présente invention possèdent une capacité d'incorporation de gènes d'intérêts améliorée, résultant de l'inactivation par délétion du gène IVa2; et, surtout, sont nettement moins immunogènes puisque l'expression des gènes tardifs éventuellement présents dans les adénovirus recombinants selon l'invention est considérablement diminuée, voire abolie.

Les vecteurs de l'invention sont donc particulièrement avantageux puisqu'ils permettent l'incorporation de gènes d'intérêt de grande taille (supérieure à 8 kb et pouvant atteindre plus de 11 kb) sans particulièrement affecter les titres obtenus, et qu'ils possèdent très peu de régions virales exprimées, ce qui réduit fortement voire supprime les risques inhérents à l'utilisation de virus comme vecteurs en thérapie génique tels que l'immunogénicité, la pathogénicité, la transmission, la réplication, la recombinaison, etc. La présente invention fournit ainsi des vecteurs viraux particulièrement adaptés au transfert et à l'expression in vivo de séquences d'ADN désirées.

Un premier objet de la présente invention concerne donc un adénovirus recombinant défectif dans lequel le gène IVa2 au moins est inactivé.

Les adénovirus sont dits défectifs lorsqu'ils sont incapables de se répliquer de façon autonome dans les cellules infectées. Généralement, le génome des adénovirus selon la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par une séquence d'acides nucléiques hétérologue.
Comme indiqué ci-avant, la demanderesse a maintenant montré qu'il est possible d'inactiver le gène IVa2 dans le génome de l'adénovirus sans affecter les propriétés recherchées de ce virus. Plus particulièrement, pour la préparation des vecteurs de l'invention, le gène IVa2 peut être inactivé par différentes techniques connues de l'homme du métier, et notamment par suppression, substitution, délétion, et/ou addition d'une ou plusieurs bases. De telles modifications peuvent être obtenues in vitro (sur de l'ADN isolé) ou in situ, par exemple, au moyen des techniques du génie génétique, ou encore par traitement au moyen d'agents mutagènes. La ou lesdites modifications génétiques peuvent être localisées dans la partie codante du gène, ou en dehors de la région codante, et par exemple dans les régions responsables de l'expression et/ou de la régulation transcriptionelle desdits gènes. L'inactivation du gène peut donc se manifester par la production d'une protéine inactive en raison de modifications structurales ou conformationelles, par l'absence de production, par la production d'une protéine ayant une activité altérée, ou encore par la production de la protéine naturelle à un niveau atténué ou selon un mode de régulation désiré.
Parmi les agents mutagènes utilisables pour l'inactivation, on peut citer par exemple les agents physiques tels que les rayonnements énergétiques (rayons X, γ, ultra violet, etc..), ou les agents chimiques capables de réagir avec différents groupements fonctionnels des bases de l'ADN, et par exemple les agents alkylants [éthylméthane sulfonate (EMS), N-méthyl-N'-nitro-N-nitrosoguanidine, N-nitroquinoléine-1-oxyde (NQO)], les agents bialkylants, les agents intercalants, etc.
Les modifications génétiques peuvent également être obtenues par disruption génique, par exemple selon le protocole initialement décrit par Rothstein [Meth. Enzymol. 101 (1983) 202]. Dans ce cas, tout ou partie de la séquence codante est préférentiellement perturbée pour permettre le remplacement, par recombinaison homologue, de la séquence génomique par une séquence non fonctionnelle ou mutante.

Préférentiellement, dans les adénovirus de l'invention, le gène IVa2 est inactivé par mutation et/ou délétion d'une ou de plusieurs bases. Encore plus préférentiellement, le gène IVa2 est inactivé par délétion totale ou partielle.
Plus particulièrement, on entend par délétion toute suppression de tout ou partie du gène considéré. Il peut s'agir notamment de tout ou partie de la région codante dudit gène, et/ou de tout ou partie de la région promotrice de la transcription dudit gène. La suppression peut être effectuée par digestion au moyen d'enzymes de restriction appropriées, puis ligature, selon les techniques classiques de biologie moléculaire, ainsi qu'illustré dans les exemples.
De manière particulièrement préférée, l'inactivation des gènes est réalisée de telle sorte qu'elle n'affecte que le gène considéré et pas les autres gènes viraux, notamment les gènes voisins. Par ailleurs, certaines altérations telles que des mutations ponctuelles étant par nature capables d'être corrigées ou atténuées par des mécanismes cellulaires, il est particulièrement préféré que l'inactivation soit parfaitement stable ségrégationellement et/ou non-réversible.
Selon un mode particulièrement avantageux, dans les adénovirus recombinants de la présente invention, le gène IVa2 est inactivé par délétion d'un fragment BssHII-BstEII allant du nucléotide 4106 au nucléotide 5186, sur la séquence de l'adénovirus Ad5. Cette séquence est accessible dans la littérature et également sur base de données (voir notamment Genebank n° M73260).

Les adénovirus recombinants selon l'invention comprennent avantageusement les séquences ITR et une région permettant l'encapsidation.

Les séquences inversées répétées (ITR) constituent l'origine de réplication des adénovirus. Elles sont localisées aux extrémités 3' et 5' du génome viral (Cf figure 1), d'où elles peuvent être isolées aisément selon les techniques classiques de biologie moléculaire connues de l'homme du métier. La séquence nucléotidique des séquences ITR des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence ITR gauche correspond à la région comprenant les nucléotides 1 à 103 du génome.
La séquence d'encapsidation (également désignée séquence Psi) est nécessaire à l'encapsidation du génome viral. Cette région doit donc être présente pour permettre la préparation d'adénovirus recombinants défectifs selon l'invention. La séquence d'encapsidation est localisée dans le génome des adénovirus, entre l'ITR gauche (5') et le gène E1 (Cf figure 1). Elle peut être isolée ou synthétisée artificiellement par les techniques classiques de biologie moléculaire. La séquence nucléotidiques de la séquence d'encapsidation des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, une séquence d'encapsidation fonctionnelle est comprise entre les nucléotides 194 et 358 du génome.

Dans un premier mode particulier de réalisation, les adénovirus recombinants de l'invention portent une délétion de tout ou partie des gènes E1 et IVa2.
Dans un autre mode particulier de réalisation, les adénovirus recombinants de l'invention portent une délétion de tout ou partie des gènes E4 et IVa2.
Toujours selon un mode préféré de réalisation, les adénovirus recombinants de l'invention portent une délétion de tout ou partie des gènes E1, IVa2 et E3.
Dans une variante particulièrement avantageuse, les adénovirus recombinants de l'invention portent une délétion de tout ou partie des gènes E1, IVa2 et E4.

Des adénovirus recombinants selon l'invention possédant des propriétés particulièrement attractives pour une utilisation en thérapie génique sont ceux portant une délétion de tout ou partie des gènes E1, IVa2, E3, E4 et éventuellement L5.Ces vecteurs combinent en effet des propriétés d'infection, de sécurité et de capacité de transfert de gènes très élevées.

Avantageusement, les adénovirus recombinants de l'invention comportent en outre une séquence d'acides nucléiques hétérologue dont le transfert et/ou l'expression dans une cellule, un organe ou un organisme est recherché.

En particulier, la séquence d'ADN hétérologue peut comporter un ou plusieurs gènes thérapeutiques et/ou un ou plusieurs gènes codant pour des peptides antigéniques.
Les gènes thérapeutiques qui peuvent ainsi être transférés sont tout gène dont la transcription et éventuellement la traduction dans la cellule cible génèrent des produits ayant un effet thérapeutique.
Il peut s'agir en particulier de gènes codant pour des produits protéiques ayant un effet thérapeutique. Le produit protéique ainsi codé peut être une protéine, un peptide, un acide aminé, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule lui permettant de lutter contre une pathologie.
Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc, les gènes suicides : Thymidine kinase, cytosine désaminase, etc.

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Comme indiqué plus haut, la séquence d'ADN hétérologue peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, la séquence d'acides nucléiques hétérologue comprend également une région promotrice de la transcription fonctionnelle dans la cellule infectée. II peut s'agir d'une région promotrice naturellement responsable de l'expression du gène considéré lorsque celle-ci est susceptible de fonctionner dans la cellule infectée. Il peut également s'agir de régions d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces régions promotrices peuvent être modifiées par addition de séquences d'activation, de régulation, ou permettant une expression tissu-spécifique ou majoritaire. Par ailleurs, lorsque l'acide nucléique hétérologue ne comporte pas de séquences promotrices, il peut être inséré dans le génome du virus défectif en aval d'une telle séquence.
Par ailleurs, la séquence d'acides nucléiques hétérologue peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Toujours dans un mode particulierement avantageux, les vecteurs de l'invention possèdent en outre un gène E3 fonctionnel sous controle d'un promoteur hétérologue. Plus préférentiellement, les vecteurs possèdent une partie du gène E3 permettant l'expression de la protéine gp19K. Cette protéine permet en effet d'éviter que le vecteur adénovirus fasse l'objet d'une réaction immunitaire qui (i) limiterait son action et (ii) pourrait avoir des effets secondaires indésirables.

Les adénovirus recombinants selon l'invention peuvent être d'origines diverses. Il existe en effet différents sérotypes d'adénovirus, dont la structure et les propriétés varient quelque peu, mais qui présentent une organisation génétique comparable. De ce fait, les enseignements décrits dans la présente demande peuvent être aisément reproduits par l'homme du métier pour tout type d'adénovirus.

Plus particulièrement, les adénovirus de l'invention peuvent être d'origine humaine, animale, ou mixte (humaine et animale).
Concernant les adénovirus d'origine humaine, on préfère utiliser ceux classés dans le groupe C. Plus préférentiellement, parmi les différents sérotypes d'adénovirus humain, on préfère utiliser dans le cadre de la présente invention les adénovirus de type 2 ou 5 (Ad 2 ou Ad 5).

Comme indiqué plus haut, les adénovirus de l'invention peuvent également être d'origine animale, ou comporter des séquences issues d'adénovirus d'origine animale. La demanderesse a en effet montré que les adénovirus d'origine animale sont capables d'infecter avec une grande efficacité les cellules humaines, et qu'ils sont incapables de se propager dans les cellules humaines dans lesquelles ils ont été testés (Cf demande FR 93 05954). La demanderesse a également montré que les adénovirus d'origine animale ne sont nullement trans-complémentés par des adénovirus d'origine humaine, ce qui élimine tout risque de recombinaison et de propagation in vivo, en présence d'un adénovirus humain, pouvant conduire à la formation d'une particule infectieuse. L'utilisation d'adénovirus ou de régions d'adénovirus d'origine animale est donc particulièrement avantageuse puisque les risques inhérents à l'utilisation de virus comme vecteurs en thérapie génique sont encore plus faibles.
Les adénovirus d'origine animale utilisables dans le cadre de la présente invention peuvent être d'origine canine, bovine, murine, (exemple : Mavl, Beard et al., Virology 75 (1990) 81), ovine, porcine, aviaire ou encore simienne (exemple : SAV). Plus particulièrement, parmi les adénovirus aviaires, on peut citer les sérotypes 1 à 10 accessibles à l'ATCC, comme par exemple les souches Phelps (ATCC VR-432), Fontes (ATCC VR-280), P7-A (ATCC VR-827), IBH-2A (ATCC VR-828), J2-A (ATCC VR-829), T8-A (ATCC VR-830), K-11 (ATCC VR-921) ou encore les souches référencées ATCC VR-831 à 835. Parmi les adénovirus bovins, on peut utiliser les différents sérotypes connus, et notamment ceux disponibles à l'ATCC (types 1 à 8) sous les références ATCC VR-313, 314, 639-642, 768 et 769. On peut également citer les adénovirus murins FL (ATCC VR-550) et E20308 (ATCC VR-528), l'adénovirus ovin type 5 (ATCC VR-1343), ou type 6 (ATCC VR-1340); l'adénovirus porcin 5359), ou les adénovirus simiens tels que notamment les adénovirus référencée à l'ATCC sous les numéros VR-591-594, 941-943, 195-203, etc.
De préférence, parmi les différents adénovirus d'origine animale, on utilise dans le cadre de l'invention des adénovirus ou des régions d'adénovirus d'origine canine, et notamment toutes les souches des adénovirus CAV2 [souche manhattan ou A26/61 (ATCC VR-800) par exemple]. Les adénovirus canins ont fait l'objet de nombreuses études structurales. Ainsi, des cartes de restriction complètes des adénovirus CAV1 et CAV2 ont été décrites dans l'art antérieur (Spibey et al., J. Gen. Virol. 70 (1989) 165), et les gènes E1a, E3 ainsi que les séquences ITR ont été clonés et séquencés (voir notamment Spibey et al., Virus Res. 14 (1989) 241; Linné, Virus Res. 23 (1992) 119, WO 91/11525).

Les adénovirus recombinants défectifs selon l'invention peuvent être préparés de différentes façons.
Une première méthode consiste à transfecter l'ADN du virus recombinant défectif préparé in vitro (soit par ligature, soit sous forme de plasmide) dans une lignée cellulaire compétente, c'est-à-dire portant en trans toutes les fonctions nécessaires à la complémentation du virus défectif Ces fonctions sont préférentiellement intégrées dans le génome de la cellule, ce qui réduit les risques de recombinaison, et confère une stabilité accrue à la lignée cellulaire. La préparation de telles lignées cellulaires est décrite dans les exemples.
Une seconde approche consiste à co-transfecter dans une lignée cellulaire apppropriée l'ADN du virus recombinant défectif préparé in vitro (soit par ligature, soit sous forme de plasmide) et l'ADN d'un ou de plusieurs virus ou plasmide helper. Selon cette méthode; il n'est pas nécessaire de disposer d'une lignée cellulaire compétente capable de complémenter toutes les fonctions défectives de l'adénovirus recombinant. Une partie de ces fonctions est en effet complémentée par le ou les virus helper. Ce ou ces virus helper sont eux-mêmes défectifs. La préparation d'adénovirus recombinants défectifs de l'invention selon cette méthode est également illustrée dans les exemples.
A cet égard, la présente demande décrit également la construction de plasmides portant la partie gauche modifiée du génome de l'adénovirus Ad5 (plasmides pCO1 et pCO2). Ces plasmides sont particulièrement utiles pour la construction d'adénovirus recombinants défectifs en tant que vecteurs de thérapie génique. Ainsi, le plasmide pCO1 porte la région gauche du génome de l'adénovirus, depuis l'ITR gauche jusqu'au nucléotide 6316, avec une délétion de la région comprise entre les nucléotides 382-3446, correspondant au gène E1. Ce plasmide est particulièrement avantageux puisqu'il comporte, par rapport aux autres plasmide de ce type décrits dans l'art antérieur, une délétion plus grande au niveau du gène E1, offrant plus de capacités de clonage et, surtout, moins de risques de recombinaison. Le plasmide pCO2 dérive du plasmide pCO1 par délétion d'une région supplémentaire comprise entre les nucléotides 4106-5186, et correspondant à une partie du gène IVa2. Cette délétion a l'avantage de ne pas affecter le gène E2. Le plasmide pCO6 dérive du plasmide pCO1 par insertion d'un codon stop dans la phase de lecture du gèneIVa2. Les plasmides pCO1, pCO2 et pCO6 contiennent par ailleurs un multisite de clonage permettant l'incorporation d'une séquence d'acides nucléiques hétérologue d'intérêt. La construction résultante peut ensuite être utilisée pour préparer l'adénovirus recombinant défectif par cotransfection avec un ADN correspondant à la partie droite du génome de l'adénovirus dans une lignée cellulaire compétente. En ce qui concerne ce dernier, il peut être issu du génome d'un virus sauvage, ou d'un virus lui-même défectif tel que Addl324 qui est délété de la région E3 : Add1808 qui est délété de la région E4 (Weinberg et al., J. Virol. 57 (1986) 833), etc (Cf exemples). Parmi les lignées cellulaires utilisables, on peut citer notamment la lignée de rein embryonnaire humain 293, les cellules KB, les cellules Hela, MDCK, GHK, etc, et plus généralement, toute lignée cellulaire complémentant pour la ou les régions délétées (Cf exemples).
Ensuite, les virus recombinants qui se sont multipliés sont récupérés, purifiés et amplifiés selon les techniques classiques de la virologie.
Le plasmide pCO1 permet ainsi la construction d'adénovirus recombinants défectifs portant une délétion au niveau du gène E1 allant du nucléotide 382 au nucléotide 3446.
Le plasmide pCO2 permet la construction d'adénovirus recombinants défectifs portant une délétion au niveau du gène E1, allant du nucléotide 382 au nucléotide 3446, et une délétion au niveau du gène IVa2, allant du nucléotide 4106 au nucléotide 5186.
Le plasmide pCO6 permet la construction d'adénovirus recombinants défectifs portant une délétion au niveau du gène E1, allant du nucléotide 382 au nucléotide 3446, et dont le gène IVa2 a été inactivé par insertion d'un codon stop dans sa phase de lecture, au niveau du site Sph1 correspondant à la base 5141 de l'adénovirus Ad5.

La présente invention décrit également une série de plasmides permettant la construction de lignées cellulaires transcomplémentant les adénovirus recombinants défectifs. En particulier le plasmide pAC5 qui possède le gène IVa2 sous contrôle de son propre promoteur et les plasmides pGY37-TK-IVa2 et pGY37-CMV-IVa2 qui portent le gène IVa2 sous contrôle respectivement des promoteurs TK et CMV. Ces plasmides portent également les séquences EBNA1/OriP qui leur permettent de se répliquer dans les cellules eucaryotes.Ces plasmides permettent donc la construction de lignées cellulaires qui transcomplémentent les adénovirus recombinant défectifs pour le gène Iva2 sans qu'il soit besoin d'utiliser de virus helper.
La présente invention concerne également toute composition pharmaceutique comprenant un ou plusieurs adénovirus recombinants défectifs tels que décrits précédemment. Les compositions pharmaceutiques de l'invention peuvent être formulées en vue d'une administration par voie topique, orale, parentérale, intranasale, intraveineuse, intramusculaire, sous-cutanée, intraoculaire, transdermique, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution de virus, et est déterminé par infection d'une culture cellulaire appropriée, et mesure, généralement après 5 jours, du nombre de plages de cellules infectées. Les techniques de détermination du titre pfu d'une solution virale sont bien documentées dans la littérature.

Selon la séquence d'ADN hétérologue insérée, les adénovirus de l'invention peuvent être utilisés pour le traitement ou la prévention de nombreuses pathologies, incluant les maladies génétiques (dystrophie, fibrose cystique, etc), les maladies neurodégénératives (alzheimer, parkinson, ALS, etc), les cancers, les pathologies liées aux désordres de la coagulation ou aux dyslipoprotéinémies, les pathologies liées aux infections virales (hépatites, SIDA, etc), etc.

La présente invention sera plus complètement décrite à l'aide des exemples qui suivent, qui doivent être considérés comme illustratifs et non limitatifs.

### Légende des figures.

Figure 1 : Organisation génétique de l'adénovirus Ad5. La séquence complète de l'Ad5 est disponible sur base de données et permet à l'homme du métier de sélectionner ou de créer tout site de restriction, et ainsi d'isoler toute région du génome.

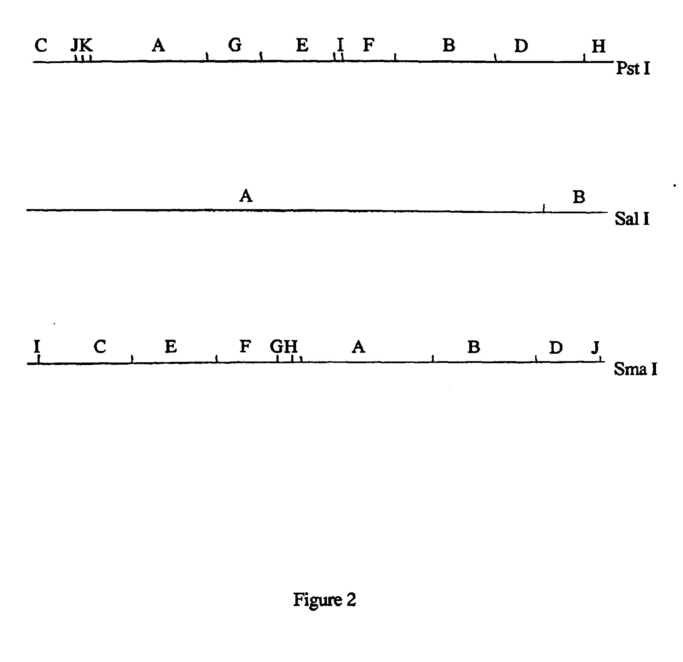

Figure 2 : Carte de restriction de l'adénovirus CAV2 souche Manhattan (d'après Spibey et al précité).
Figure 3: Carte de restriction du fragment HindIII contenu dans le plasmide pCO1.
Figure 4: Carte de restriction du fragment HindIII contenu dans le plasmide pCO2.
Figure 5: Construction et représentation du plasmide pPY32.
Figure 6: Représentation du plasmide pPY55.
Figure 7: Construction du plasmide pE4Gal.
Figure 8: Carte de restriction du fragment Hind3 contenu dans le plasmide pCO6.
Figure 9: Carte de restriction du plasmide pAC2.
Figure 10: Carte de restriction du plasmide pAC5.
Figure 11: Carte de restriction du plasmide pGY37-TK-IVa2.
Figure 12: Carte de restriction du plasmide pGY37-CMV-IVa2.

### Techniques générales de biologie moléculaire.

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].
Les plasmides de type pBR322, pUC et les phages de la série M13 sont d'origine commerciale (Bethesda Research Laboratories).
Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.
Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.
La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.
L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Lignées cellulaires utilisées.

Dans les exemples qui suivent, les lignées cellulaires suivantes ont ou peuvent être utilisées :
- Lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %).
- Lignée de cellules humaines KB : Issue d'un carcinome épidermique humain, cette lignée est accessible à l'ATCC (ref. CCL17) ainsi que les conditions permettant sa culture.
- Lignée de cellules humaines Hela : Issue d'un carcinome de l'épithelium humain, cette lignée est accessible à l'ATCC (ref. CCL2) ainsi que les conditions permettant sa culture.
- Lignée de cellules canines MDCK : Les conditions de culture des cellules MDCK ont été décrites notamment par Macatney et al., Science 44 (1988) 9.
- Lignée de cellules gm DBP6 (Brough et al., Virology 190 (1992) 624). Cette lignée est constituée de cellules Hela portant le gène E2 d'adénovirus sous le controle du LTR de MMTV.

### EXEMPLES.

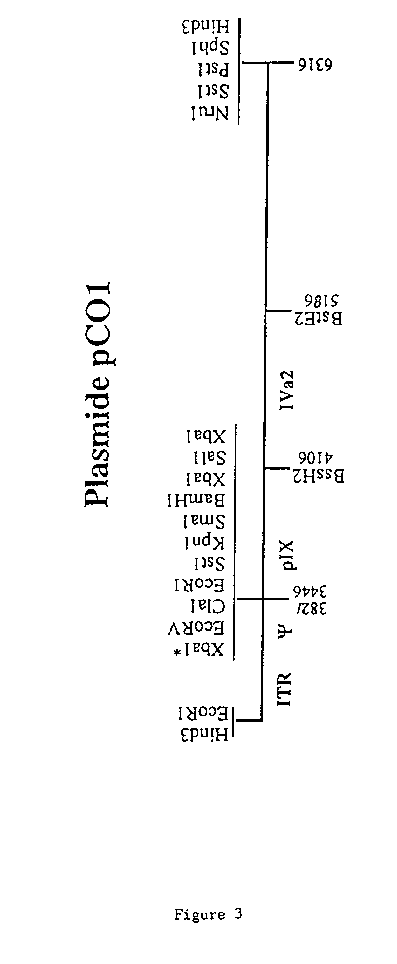

### Exemple 1 - Construction du plasmide pCO1 (figure 3).

### A - Construction du plasmide pCE.

Le fragment EcoRI-XbaI correspondant à l'extrémité gauche du génome de l'adénovirus Ad5 a d'abord été cloné entre les sites EcoRI et XbaI du vecteur pIC19H. Ceci génère le plasmide pCA. Le plasmide pCA a ensuite été coupé par HinfI, ses extrémintés 5' proéminentes ont été remplies par le fragment de klenow de l'ADN polymérase I de E.coli, puis il a été coupé par EcoRI. Le fragment ainsi généré du plasmide pCA qui contient l'extrémité gauche du génome de l'adénovirus Ad5 a ensuite été cloné entre les sites EcoRI et SmaI du vecteur pIC20H (Marsh et al., Gene 32 (1984) 481). Ceci génère le plasmide pCB. Le plasmide pCB a ensuite été coupé par EcoRI, ses extrémintés 5' proéminentes ont été remplies par le fragment de klenow de l'ADN polymérase I de E.coli, puis il a été coupé par BamHI. Le fragment ainsi généré du plasmide pCB qui contient l'extrémité gauche du génome de l'adénovirus Ad5 a ensuite été cloné entre les sites NruI et BgIII du vecteur pIC20H. Ceci génère le plasmide pCE dont une caractéristique intéressante est qu'il possède les 382 premières paires de bases de l'adénovirus Ad5 suivies d'un multisite de clonage.

### B - Construction du plasmide pCD'.

Le fragment Sau3A (3346) - SstI (3645) et le fragment SstI (3645) - NarI (5519) du génome de l'adénovirus Ad5 ont tout d'abord été ligaturés et clonés entre les sites ClaI et BamHI du vecteur pIC20H, ce qui génère le plasmide pPY53. Le fragment SalI-TaqI du plasmide pPY53 préparé à partir d'un contexte dam-, contenant la partie du génome de l'adénovirus Ad5 comprise entre les sites Sau3A (3346) et TaqI (5207) a ensuite été cloné entre les sites SalI et ClaI du vecteur pIC20H, ce qui génère le plasmide pCA'. Le fragment TaqI (5207) - NarI (5519) du génome de l'adénovirus Ad5 préparé à partir d'un contexte dam- et le fragment SalI-TaqI du plasmide pCA' ont ensuite été ligaturés et clonés entre les sites SalI et NarI du vecteur pIC20H. Ceci génère le plasmide pCC'. Le fragment NarI (5519) - NruI (6316) du génome de l'adénovirus Ad5 préparé à partir d'un contexte dam- et le fragment SalI-NarI du plasmide pCC' ont ensuite été ligaturés et clonés entre les sites SalI et NruI du vecteur pIC20R. Ceci génère le plasmide pCD'.

### C - Construction du plasmide pC01.

Une digestion partielle par XhoI puis une digestion complète par SalI du plasmide pCD' génère un fragment de restriction qui contient la séquence de l'adénovirus Ad5, du site Sau3A (3446) au site NruI (6316). Ce fragment a été cloné dans le site SalI du plasmide pCE. Ceci génère le plasmide pC01 (figure 3), qui contient la partie gauche de l'adénovirus Ad5 jusqu'au site Hinfl (382), un multisite de clonage et le fragment Sau3A (3446) - Nrul (6316) de l'adénovirus Ad5.

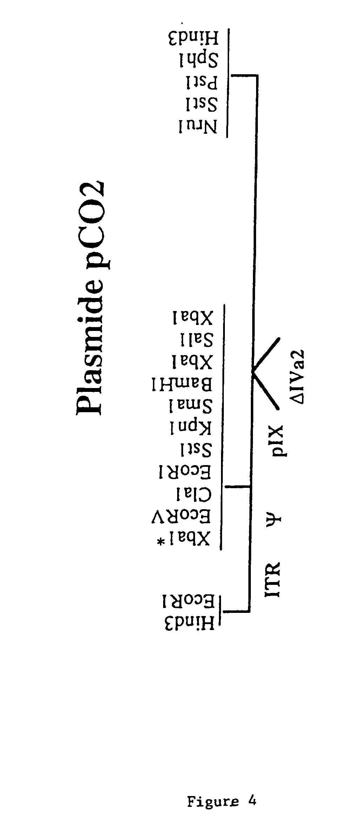

### Exemple 2 - Construction du plasmide pCO2 (figure 4).

### A - Construction du plasmide pCD".

Le fragment BssHII-BstEII correspondant au fragment BssHII (4106) - BstEII (5186) du génome de l'adénovirus Ad5 a d'abord été enlevé du plasmide pCA'. Ceci génère le plasmide pCB", dont une caractéristique intéressante est qu'il possède une délétion d'une partie de la séquence du gène IVa2. Le fragment TaqI (5207) - NarI (5519) du génome de l'adénovirus Ad5 préparé à partir d'un contexte dam- et le fragment SalI-TaqI du plasmide pCB" ont ensuite été ligaturés et clonés entre les sites SalI et NarI du vecteur pIC20H. Ceci génère le plasmide pCC". Le fragment NarI (5519) - NruI (6316) du génome de l'adénovirus Ad5 préparé à partir d'un contexte dam- et le fragment SalI-NarI du plasmide pCC" ont ensuite été ligaturés et clonés entre les sites SalI et NruI du vecteur pIC20R. Ceci génère le plasmide pCD".

### B - Construction du plasmide pC02.

Une digestion partielle par XhoI puis une digestion complète par SalI du plasmide pCD" génère un fragment de restriction qui contient la séquence de l'adénovirus Ad5, du site Sau3A (3446) au site NruI (6316), dont le fragment BssHII (4106) -BstEII (5186) a été délété. Ce fragment a été cloné dans le site SalI du plasmide pCE. Ceci génère le plasmide pC02 (figure 4), qui contient la partie gauche de l'adénovirus Ad5 jusqu'au site Hinfl (382), un multisite de clonage et le fragment Sau3A (3446) - NruI (6316) de l'adénovirus Ad5, dont le fragment BssHII (4106) - BstEII (5186) a été délété.

### Exemple 3 - Construction d'un adénovirus recombinant portant une délétion dans le gène E1.

Cet exemple décrit la construction d'un adénovirus recombinant défectif portant une délétion dans la région E1 allant du nucléotide 382 au nucléotide 3446. Cet adénovirus est particulièrement avantageux puisqu'il comporte une délétion plus grande au niveau du gène E1, offrant plus de capacités de clonage et, surtout, moins de risques de recombinaison.

Cet adénovirus recombinant a été obtenu par recombinaison in vivo, par cotransfection dans les cellules 293, en présence de phosphate de calcium, de l'ADN du virus AdRSVf3ga1 digéré par Clal et du plasmide pCO1 digéré par XmnI. Les cellules sont ensuite récoltées, disruptées par trois cycles de congélation-décongélation dans leur surnageant, puis centrifugées à 4000 rpm pendant 10 minutes. Le surnageant ainsi obtenu est ensuite amplifié sur culture cellulaire fraiche. Les virus sont alors purifiés à partir de plages et leur ADN est analysé selon la méthode de Hirt (précité). Des stocks de virus sont ensuite préparés sur gradient de chlorure de césium.

### Exemple 4 - Construction d'un adénovirus recombinant portant une délétion dans les gènes E1 et IVa2.

Cet exemple décrit la construction d'un adénovirus recombinant défectif portant une délétion dans la région E1, allant du nucléotide 382 au nucléotide 3446, plus une délétion dans la région IVa2. Cet adénovirus est particulièrement avantageux puisque, par rapport à l'adénovirus décrit dans l'exemple 3, il comporte une délétion au niveau du gène IVa2, offrant plus de capacités de clonage et, surtout, moins de risques de production de protéines virales in vivo.

### A- Construction de l'adénovirus recombinant défectif ΔE1-Δ1Va2 dans des lignées cellulaires transcomplémentant la fonction E1 (E1+),

L'adénovirus recombinant a été préparé selon les cinq protocoles suivants :
(a) l'ADN du virus AdRSVβgal digéré par ClaI et le plasmide pCO2 digéré par XmnI ont été co-transfectés dans les cellules E1⁺ (293 ou 293 E4) en présence de phosphate de calcium, pour permettre la recombinaison in vivo.
(b) l'ADN du virus AdRSVβgal digéré par ClaI et par XcaI et le plasmide pCO2 digéré par XmnI ont été co-transfectés dans les cellules E1⁺ (293 ou 293 E4) en présence de phosphate de calcium, pour permettre la recombinaison in vivo.
(c) l'ADN du virus AdRSVβga1 et le plasmide pCO2 tous deux digérés par XcaI sont d'abord ligaturés in vitro puis la construction résultante est transfectée dans les cellules E1⁺ (293 ou 293 E4) en présence de phosphate de calcium.
(d) l'ADN du virus AdRSVβgal digéré par Clal, l'ADN du plasmide pCO2 digéré par Xmn1 et l'ADN du virus helper pAC2 ont été cotransfectés dans des cellules E1⁺ (293 ou 293 E4) en présence de phosphate de calcium.
(e) l'ADN du virus AdRSVβgal digéré par Clal, l'ADN du plasmide pC06 digéré par Xmn1 et l'ADN du virus helper pAC2 ont été cotransfectés dans des cellules E1⁺ (293 ou 293 E4) en présence de phosphate de calcium.
(Les plasmides pAC2 et pC06 sont décrits dans l'exemple 7)

Les virus produits sont ensuite amplifiés et purifiés comme dans l'exemple 3.

### B- Construction d'un adénovirus ΔE1-ΔIVa2 dans des lignées cellulaires transcomplémentant la fonction IVa2.

Dans les clones cellulaires exprimant la région IVa2 de l'adénovirus Ad5, l'adénovirus ΔE1-ΔIVa2 a pu être préparé selon les deux protocoles suivants:
(a) l'ADN du virus AdRSVβgal digéré par Clal et l'ADN du plasmide pCO2 digéré par Xmn1 ont été cotransfectés dans les cellules en présence de phosphate de calcium.
(b) l'ADN du virus AdRSVβgal digéré par Cla1 et l'ADN du plasmide pCO6 digéré par Xmn1 ont été cotransfectés dans les cellules en présence de phosphate de calcium

### Exemple 5 - Construction d'un adénovirus recombinant portant une délétion dans les gènes E1, E3 et IVa2.

Cet exemple décrit la construction d'un adénovirus recombinant défectif portant une délétion dans la région E1, allant du nucléotide 382 au nucléotide 3446, une délétion dans le gène IVa2, et une délétion dans la région E3. Cet adénovirus est particulièrement avantageux puisque, par rapport à l'adénovirus décrit dans l'exemple 4, il comporte une délétion au niveau de la région E3, offrant plus de capacités de clonage.

L'adénovirus recombinant a été préparé selon les trois protocoles suivants :
(a) l'ADN du virus Add1324 digéré par Cial et le plasmide pCO2 digéré par XmnI ont été co-transfectés dans les cellules 293 en présence de phosphate de calcium, pour permettre la recombinaison in vivo.
(b) l'ADN du virus Add1324 digéré par ClaI et par XcaI et le plasmide pCO2 digéré par XmnI ont été co-transfectés dans les cellules 293 en présence de phosphate de calcium, pour permettre la recombinaison in vivo.
(c) l'ADN du virus Addl324 et le plasmide pCO2 tous deux digérés par XcaI sont d'abord ligaturés in vitro puis la construction résultante est transfectée dans les cellules 293 en présence de phosphate de calcium.

Les virus produits sont ensuite amplifiés et purifiés comme dans l'exemple 3.

### Exemple 6 - Construction d'un adénovirus recombinant portant une délétion dans les gènes E1, E3, E4 et IVa2.

Cet exemple décrit la préparation d'adénovirus recombinants défectifs selon l'invention dont le génome est délété des gènes IVa2, E1, E3 et E4. Ces adénovirus possèdent tout d'abord une capacité d'incorporation de gènes hétérologues importante. Par ailleurs, ces vecteurs présentent une sécurité élevée en raison de la délétion de la région IVa2 et de la région E4. Cette dernière est en effet impliquée dans la régulation de l'expression des gènes tardifs, dans le processing des ARN pré-messagers tardifs, dans l'extinction de l'expression des protéines de la cellule hôte et dans l'efficacité de la réplication de l'ADN viral. Ces vecteurs possèdent donc un bruit de fond de transcription et une expression de gènes viraux très réduits. Enfin, de manière particulièrement avantageuse, ces vecteurs peuvent être produits à des titres élevés.

La partie droite d'un génome viral, délété pour les régions E3 et E4 (cf. section B), ou seulement pour la région E4 comme dans le cas des virus d1808, dl1004, dl1007 ou dl1010 décrits par G. Ketner par exemple (J. of Virology, 63 (1989) 631). Selon une autre alternative, la partie droite peut contenir des délétions dans plusieurs régions et par exemple dans les régions E3 et E4 comme celles contruites in vitro et présentées dans le virus préparé comme suit à partir du plasmide pPY55.

### A/ Construction de l'adénovirus délété dans les gènes E1, E3 et E4.

### A.1 Construction du plasmide pPY55.

### a) Construction du plasmide pPY32.

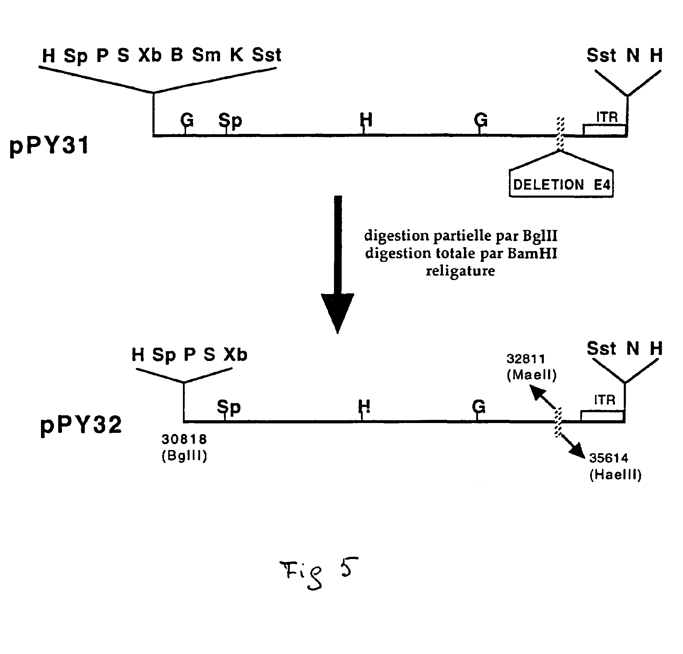

Le fragment AvrII-BcII du plasmide pFG144 [F.L. Graham et al. EMBO J. 8 (1989) 2077-2085], correspondant à l'extrémité droite du génome de l'adénovirus Ad5, a d'abord été cloné entre les sites XbaI et BamHI du vecteur pIC19H, préparé à partir d'un contexte dam-. Ceci génère le plasmide pPY23. Une caractéristique intéressante du plasmide pPY23 est que le site SalI provenant du multisite de clonage du vecteur pIC19H reste unique et qu'il est localisé à coté de l'extrémité droite du génome de l'adénovirus Ad5. Le fragment HaeIII-SaII du plasmide pPY23 qui contient l'extrémité droite du génome de l'adénovirus Ad5, à partir du site HaeIII localisé en position 35614, a ensuite été cloné entre les sites EcoRV et XhoI du vecteur pIC20H, ce qui génère le plasmide pPY29. Une caractéristique intéressante de ce plasmide est que les sites XbaI et ClaI provenant du multisite de clonage du vecteur pIC20H sont localisés à coté de la jonction EcoRV/HaeIII résultant du clonage. De plus cette jonction modifie le contexte nucléotidique immédiatement adjacent au site ClaI qui est maintenant devenu méthylable dans un contexte dam+. Le fragment XbaI(30470)-MaeII(32811) du génome de l'adénovirus Ad5 a ensuite été cloné entre les sites XbaI et ClaI du plasmide pPY29 préparé à partir d'un contexte dam-, ce qui génère le plasmide pPY30. Le fragment SstI du plasmide pPY30, qui correspond à la séquence du génome de l'adénovirus Ad5 du site SstI en position 30556 jusqu'à l'extrémité droite a enfin été cloné entre les sites SstI du vecteur pIC20H, ce qui génère le plasmide pPY31, dont une carte de restriction de l'insert localisé entre les sites HindIII est donnée à la Figure 5.

Le plasmide pPY32 a été obtenu après digestion partielle du plasmide pPY31 par BglII, suivie d'une digestion totale par BamHI, puis religature. Le plasmide pPY32 correspond donc à la délétion du génome de l'adénovirus Ad5 située entre le site BamHI du plasmide pPY31 et le site BglII localisé en position 30818. Une carte de restriction du fragment HindIII du plasmide pPY32 est donnée à la Figure 5. Une caractéristique du plasmide pPY32 est qu'il possède des sites SaII et XbaI uniques.

### b) Construction du plasmide pPY47.

Le fragment BamHI(21562)-XbaI(28592) du génome de l'adénovirus Ad5 a tout d'abord été cloné entre les sites BamHI et XbaI du vecteur plC19H préparé à partir d'un contexte dam-, ce qui génère le plasmide pPY17. Ce plasmide contient donc un fragment HindIII (26328)-BgIII(28133) du génome de l'adénovirus Ad5, qui peut être cloné entre les sites HindIII et BgIII du vecteur pIC20R, pour générer le plasmide pPY34. Une caractéristique de ce plasmide est que le site BamHI provenant du multisite de clonage est localisé à proximité immédiate du site HindIII(26328) du génome de l'adénovirus Ad5.

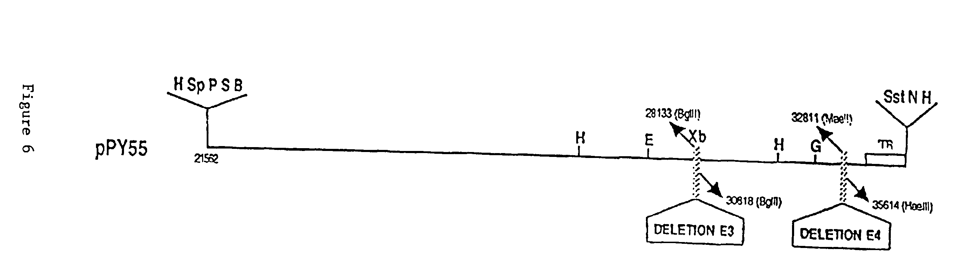

Le fragment BamHI (21562)-HindIII(26328) du génome de l'adénovirus Ad5 provenant du plasmide pPY17 a ensuite été cloné entre les sites BamHI et HindIII du plasmide pPY34, ce qui génère le plasmide pPY39. Le fragment BamHI-XbaI du plasmide pPY39 préparé à partir d'un contexte dam-, contenant la partie du génome de l'adénovirus Ad5 comprise entre les sites BamHI(21562) et BgIII(28133), a ensuite été cloné entre les sites BamHI et XbaI du vecteur pIC19H préparé à partir d'un contexte dam-. Ceci génère le plasmide pPY47 dont une caractéristique intéressante est que le site SalI provenant du multisite de clonage est localisé à proximité du site HindIII (figure 6).

### c) Construction du plasmide pPY55.

Le fragment SaII-XbaI du plasmide pPY47 préparé à partir d'un contexte dam-, et qui contient la partie du génome de l'adénovirus Ad5 allant du site BamHI(21562) jusqu'au site BglII(28133), a été cloné entre les sites SalI et XbaI du plasmide pPY32, ce qui génère le plasmide pPY55. Ce plasmide est directement utilisable pour l'obtention d'adénovirus recombinants au moins délétés pour la région E3 (délétion entre les sites BgIII localisés aux positions 28133 et 30818 du génome de l'adénovirus Ad5) et pour la région E4 dans son intégralité (délétion entre les sites MaeII(32811) et HaeIII(35614) du génome de l'adénovirus Ad5 (figure 6).

### A.2. Construction du plasmide pE4Gal.

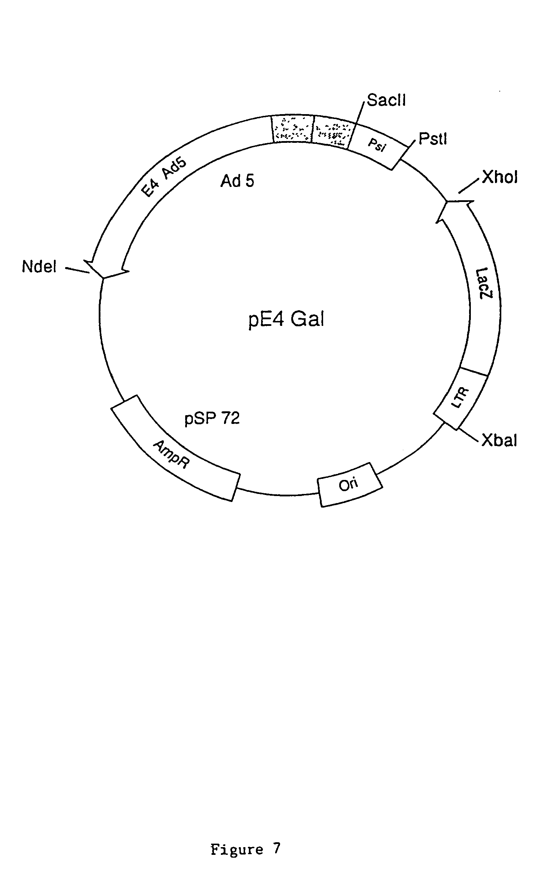

Pour cela, un plasmide portant les ITR jointives de l'Ad5, la séquence d'encapsidation, le gène E4 sous controle de son propre promoteur et, comme gène hétérologue, le gène LacZ sous controle du promoteur LTR du virus RSV a été construit (figure 7). Ce plasmide, désigné pE4Gal a été obtenu par clonage et ligature des fragments suivants (voir figure 7) :
- fragment HindIII-SacII issu du plasmide pFG144 (Graham et al, EMBO J. 8 (1989) 2077). Ce fragment porte les séquences ITR de l'Ad5 en tête à queue et la séquence d'encapsidation : fragment HindIII (34920)-SacII (352).
- fragment de l'Ad5 compris entre les sites SacII (localisé au niveau de la paire de bases 3827) et PstI (localisé au niveau de la paire de bases 4245);
- fragment de pSP 72 (Promega) compris entre les sites PstI (pb 32) et SalI (pb 34);
- fragment XhoI-XbaI du plasmide pAdLTR GaIIX décrit dans Stratford-Perricaudet et al (JCI 90 (1992) 626). Ce fragment porte le gène LacZ sous controle du LTR du virus RSV.
- fragment XbaI (pb 40) - NdeI (pb 2379) du plasmide pSP 72;
- fragment NdeI (pb 31089) - HindIII (pb 34930) de l'Ad5. Ce fragment localisé dans l'extrémité droite du génome de l'Ad5 contient la région E4 sous controle de son propre promoteur. Il a été cloné au niveau des sites NdeI (2379) du plasmide pSP 72 et HindIII du premier fragment.

Ce plasmide a été obtenu par clonage des différents fragments dans les régions indiquées du plasmide pSP 72. Il est entendu que des fragments équivalents

### A.3. Co-transfection dans les cellules 293

Les adénovirus sont obtenus recombinaison in vivo, selon les stratégies suivantes :
(i) L'ADN du virus Ad-d1324 (Thimmappaya et al., Cell 31 (1982) 543) et le plasmide pPY55, tous deux digérés par BamHI, sont d'abord ligaturés in vitro, puis cotransfectés avec le plasmide pE4Gal dans les cellules 293.
(ii) L'ADN du virus Ad-d1324 digéré par EcoRI et le plasmide pPY55 digéré par BamHI sont contranfectés, avec le plasmide pE4Gal, dans les cellules 293.
(iii) L'ADN de l'adénovirus Ad5 et le plasmide pPY55, tous deux digérés par BamHI, sont ligaturés puis cotransfectés avec le plasmide pE4Gal dans les cellules 293.
(iv) L'ADN de l'adénovirus Ad5 digéré par EcoRI et le plasmide pPY55 digéré par BamHI sont cotransfectés avec le pE4Gal dans les cellules 293.

Les stratégies (i) et (ii) permettent de générer un adénovirus recombinant délété pour les régions E1, E3 et E4; les stratégies (iii) et (iv) permettent de générer un adénovirus recombinant délété pour les régions E3 et E4. Par ailleurs, il est également possible d'utiliser une lignée cellulaire dérivée d'une lignée exprimant la région E1, par exemple la lignée 293, et exprimant également au moins les phases ouvertes ORF6 et ORF6/7 de la région E4 de l'adénovirus Ad5 (Cf FR93 08596). L'utilisation de telles lignées permet d'éviter l'emploi du plasmide pE4Gal.

### B/ Construction de l'adénovirus délété dans les gènes IVa2, E1, E3 et E4.

Cet adénovirus a été obtenu par recombinaison, après co-transfection dans les cellules 293 du plasmide pCO2 (exemple 2) et la partie droite d'un adévovirus au moins délété pour la région E4, et par exemple l'adénovirus obtenu après utilisation du plasmide pPY55 (cf. section A).
Après co-transfection au phosphate de calcium, les cellules sont récoltées, disruptées par trois cycles de congélation-décongélation dans leur surnageant, puis centrifugées à 4000 rpm pendant 10 minutes. Le surnageant ainsi obtenu est ensuite amplifié sur culture cellulaire fraiche. Les virus sont alors purifiés à partir de plages et leur ADN est analysé selon la méthode de Hirt (précité). Des stocks de virus sont ensuite préparés sur gradient de chlorure de césium.

### Exemple 7- Construction d'un adénovirus recombinant portant une délétion dans les gènes E1, E4, IVa2.

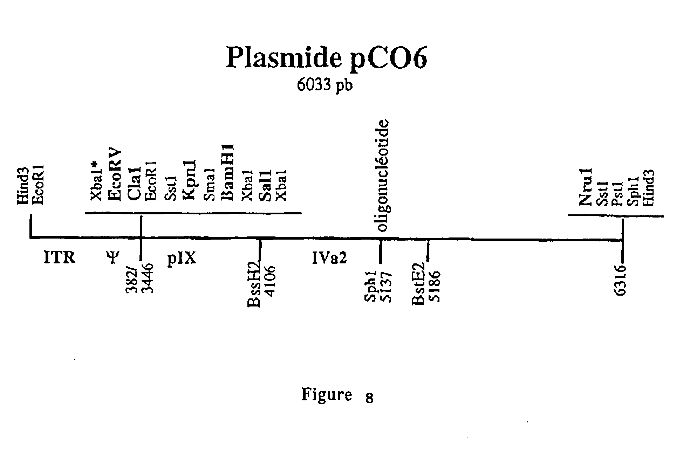

### A - Construction du plasmide pC06 (figure 8).

### A.1 - Construction du plasmide pGY38

Le fragment Munl - Nrul du plasmide pCO1 contenant les séquences du génome de l'adénovirus Ad5 allant des bases 3924 à 6316 et notamment le gène IVa2 (bases 4094 à 5719) a été cloné entre les sites Munl et Nrul du vecteur commercial pSL1180. Ceci génère le plasmide pGY38.

### A.2 - Construction du plasmide pGY39.

La séquence nucléotidique suivante et son brin complémentaire ont été synthétisées artificiellement par les techniques classiques de biologie moléculaire :
5'- CCT TAG CCC GGG CTA AGG CAT G - 3' (SEQ ID n°1)
Chacun des brin possède un site de restriction Sph1 à son extrémité 3'. Cette séquence a été clonée au site Sph1 du plasmide pGY38: elle introduit un codon stop dans la partie codante du gène IVa2, au site Sph1 correspondant à la base 5141 de l'adénovirus Ad5. Ceci génère le plasmide pGY39. Dans ce plasmide, le gène IVa2 ainsi modifié est inactivé: il code une protéine inactive correspondant aux 102 premiers acides aminés de la protéine IVa2 sauvage.

### A.3 - Construction du plasmide pCO6.

Le fragment Mun1- Nru1 du plasmide pGY39 a été cloné entre les sites Mun1 et Nrul du plasmide pCO1. Ceci génère le plasmide pC06 (figure 8) qui contient la partie gauche de l'adénovirus Ad5 jusqu'au site Hinf1 (382), un multisite de clonage, le fragment Sau3A (3446) - Sph1 (5141) de l'adénovirus Ad5, foligonucléotide apportant un codon stop dans la séquence codante du gène IVa2 et le fragment Sph1 (5141) - Nru1 (6316) de l'adénovirus Ad5.

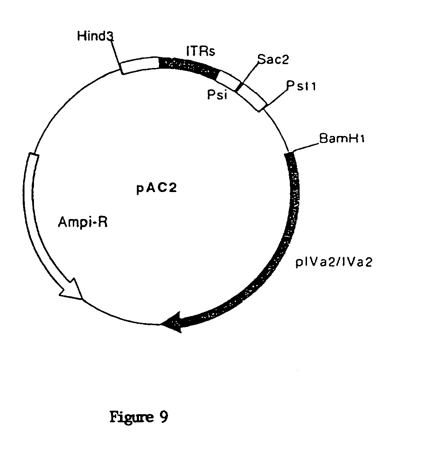

### B - Construction du plasmide pAC2 (figure 9).

### B.1- Construction du plasmide pSPITR.

Les fragments suivants ont été clonés:
- fragment Hind3 (34930) - Sac2 (357) issu du plasmide pFG144 (Graham et al, EMBO J.8, 1989, 2027), ce fragment porte les séquences ITR de l'adénovirus Ad5 en tête à queue et la séquence d'encapsidation,
- fragment de l'adénovirus Ad5 compris entre les sites Sac2 (localisé au niveau de la base 3827 dans l'Ad5) et Pst1 (localisé au niveau de la base 4245)
Puis le plasmide pSPITR a été obtenu par ligature de ces fragments entre les sites Hind3 et Pst 1 du plasmide commercial pSP72.

### B.2 - Construction du plasmide pAC2.

Le fragment Dra1 - Nru1 du plasmide pCO1, qui contient les séquences du génome de l'adénovirus Ad5 allant des bases 4029 à 6316 et notamment le gène IVa2 et son promoteur, a été cloné au site EcoRV du vecteur pIC20H (Ref. J.L. Marsh et al (1984) Gene, 32, 481-485). Ceci génère le plasmide pACl. Le fragment Bgl2 - BamHl du plasmide pACl a été cloné au site BamH1 du plasmide pSPITR, ce qui génère le plasmide pAC2 (figure 9).

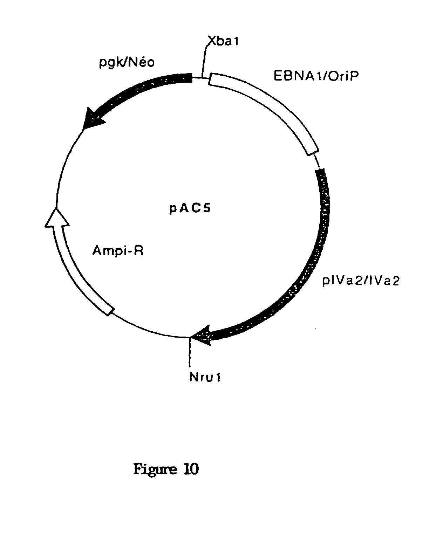

### C- Construction du plasmide pAC5 (figure 10).

### C.1- Construction du plasmide pAC3.

Le fragment EcoR1- Xba1 du plasmide commercial pMEP4 (Invitrogen) a été ligué entre les sites EcoR1 et Xba1 du plasmide pAC1. Ceci génère le plasmide pAC3 dont une caractéristique est qu'il contient les séquences EBNA1-OriP et le gène IVa2.

### C.2 - Construction du plasmide pAC5.

Le fragment Sal1 - Xho1 du plasmide commercial pMSCV a été cloné au site Sal1 du vecteur pIC20H. Ceci génère le plasmide pAC4 qui contient le gène Néo. Le fragment Xba1 - Nrul du plasmide pAC3 a été cloné entre les sites Xba1 et Nrul du plasmide pAC4. Ceci génère le plasmide pAC5 (figure 10) dont une caractéristique importante est qu'il possède le gène Néo et le gène IVa2 sous le contrôle de son propre promoteur. De plus, ce plasmide peut se répliquer dans les cellules eucaryotes car il porte les séquences EBNA1/OriP.

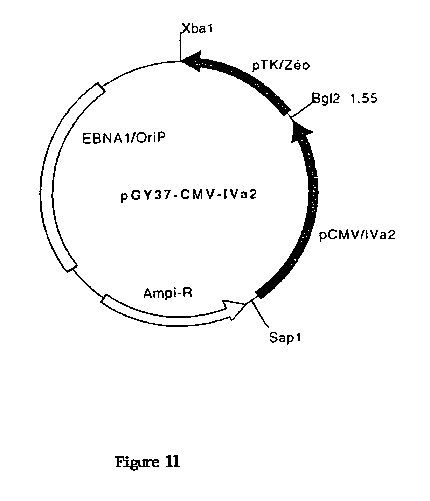

### D- Construction du plasmide pGY37-TK-IVa2 (figure 11).

### D.1 - Construction du plasmide pGY32.

La séquence nucléotidique suivante et son brin complémentaire ont été synthétisées artificiellement par les techniques classiques de biologie moléculaire :
5' - TCG ACG GAT CCC TTA AGG TTG ACG CCG CCA CCA TGG AAA CCA GAG GGC GAA GAC CGG CAG C - 3' (SEQ ID n°2)
Elle porte un site de restriction pour SalI à son extrémitée 5' et un site de restriction pour Eco47III à son extrémitée 3'. Cette séquence a été insérée dans le plasmide pAC 1 entre le site Sall et le site Eco47III du début du gène IVa2 qui correspond à la base 5411 de la séquence de l'adénovirus Ad5. Ceci génère le plasmide pGY32 dont une caractéristique importante est qu'il possède un multisite de clonage (BamHl, Afl2, Hinc2) en amont d'un consensus Kozak avant l'ATG du gène IVa2. De plus, dans cette construction, l'intron du gène IVa2 a été supprimé.

### D.2 - Construction du plasmide pGY33-TK.

Le fragment BamH1 (-110) - Hinc2 (+35) du promoteur TK du plasmide pX4B (B. Wasylyk et al, N.A.R. (1987), 15, 13, 5490) et un multisite de clonage borné par les sites Sal1 et BamH1 ont été ligaturés puis clonés entre les sites Sal1 et Hinc2 du plasmide PGY32. Ceci génère le plasmide pGY33-TK dont une caractéristique intéressante est qu'il porte le gène IVa2 dépourvu d'intron sous le contrôle du promoteur TK.

### D.3- Construction du plasmide pGY34.

Les fragments suivants ont été préparés puis ligaturés:
- le fragments Xba1 - Pvu1 du plasmide commercial pMEP4 (5.5 kb), ce fragment contient les séquences EBNA1/OriP et l'extrémité 5' du gène de résistance à l'ampicilline,
- le fragment Pvu1 - AlwN1 du plasmide commercial pMEP4 (0.8 kb), ce fragment contient l'extrémité 3' du gène de résistance à l'ampicilline et une partie de l'origine de réplication,
- le fragment Xba1 et AlwN1 du plasmide pIC20H (0.8 kb), ce fragment contient la fin de l'origine de réplication.
Ceci génère le plasmide pGY34 qui contient les séquences EBNA1/OriP.

### D.4 - Construction du plasmide pGY36.

Le fragment BamHl du plasmide commercial pUT614 (Cayla) a été cloné au site BamHl du vecteur pIC20H. Ceci génère le plasmide pPY9 qui contient le gène Zéo. Le fragment Xba - EcoRV du plasmide pPY9 a été cloné entre les sites Xbal et Nru1 du vecteur pIC20R, ce qui génère le plasmide pGY35. Le fragment Xbal du plasmide pGY35 préparé dans un contexte dam- a ensuite été cloné au site Xba1 du plasmide pGY34. Ceci génère le plasmide pGY36.

### D.5 - Construction du plasmide pGY37-TK-IVa2 (figure 11).

Le fragment Bgl2 - Sap1 du plasmide pGY33-TK a été cloné entre les sites Bgl2 et Sapl du plasmide pGY36. Ceci génère le plasmide pGY37-TK-IVa2 (figure 11) dont une caractéristique importante est qu'il possède le gène Zéo et le gène IVa2, sans intron, sous le contrôle du promoteur TK. De plus, ce plasmide peut se répliquer dans les cellules eucaryotes car il porte les séquences EBNA1/OriP.

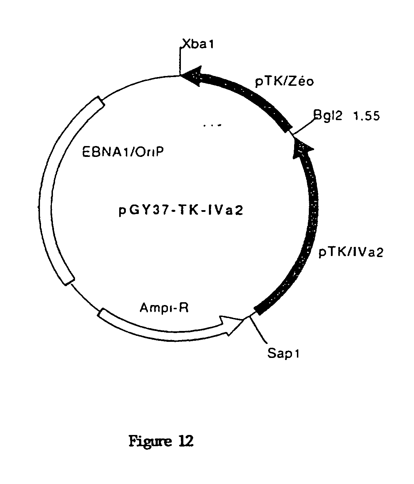

### E- Construction du plasmide pGY37-CMV-IVa2 (figure 12).

### E1- Construction du plasmide pGY33-CMV.

Le fragment Bgl2 - Afl2 du plasmide commercial pCI (Promega) a été cloné entre les sites BamH1 et Afl2 du plasmide PGY32. Ceci génère le plasmide pGY33-CMV dont une caractéristique intéressante est qu'il porte le gène IVa2 dépourvu d'intron sous le contrôle du promoteur CMV.

### E.2 - Construction du plasmide pGY37-CMV-IVa2 (figure 12).

Le fragment Bgl2 - Sapl du plasmide pGY33-CMV a été cloné entre les sites Bgl2 et Sapl du plasmide pGY36. Ceci génère le plasmide pGY37-CMV-IVa2 (figure 12) dont une caractéristique importante est qu'il possède le gène Zéo et le gène IVa2, sans intron, sous le contrôle du promoteur CMV. De plus, ce plasmide peut se répliquer dans les cellules eucaryotes car il porte les séquences EBNA1/OriP.

### F - Construction de clones cellulaires exprimant la région IVa2 de l'adénovirus Ad5.

Différents types de lignées cellulaires transcomplémentant la fonction IVa2 ont été construits dans des cellules E1⁺ (cellules 293 ou 293 E4)
(a) le plasmide pAC5 a été transfecté dans les cellules 293 en présence de phosphate de calcium, les clones cellulaires portant le plasmide réplicatif pAC5 ont été sélectionnés en présence de généticine (Sigma, 400µg/ml).
(b) le plasmide pGY37-TK-IVa2 a été transfecté dans les cellules en présence de phosphate de calcium, les clones cellulaires portant le plasmide réplicatif pGY37-TK-IVa2 ont été sélectionnés en présence de phléomycine (Cayla, 15µg/ml pour les cellules 293 et 30µg/ml pour les cellules 293 E4).
(c) le plasmide pGY37-CMV-IVa2 a été transfecté dans les cellules en présence de phosphate de calcium, les clones cellulaires portant le plasmide réplicatif pGY37-CMV-IVa2 ont été sélectionnés en présence de phléomycine (Cayla, 15µg/ml pour les cellules 293 et 30µg/ml pour les cellules 293 E4).
Plus précisément, dans chacun de ces trois cas, des cellules en boites de 5cm de diamètre ont été transfectées par 1 à 5 µg de plasmide en présence de phosphate de calcium. Après transfection des cellules, celles ci sont lavées, puis le milieu de culture (MEM, Sigma) supplémenté en sérum de veau foetal (7% final) est ajouté et les cellules sont mises à incuber pendant 24 heures. Le lendemain, on sélectionne les cellules en présence de généticine ou de phléomycine. La généticine ou la phléomycine est changée tous les trois jours et les clones sélectionnables apparaissent après environ trois semaines. Quand toutes les cellules non transfectées sont mortes, seules les cellules transfectées se divisent pour générer des clones cellulaires. Quand les clones cellulaires sont suffisamment gros pour être visibles à l'oeil nu, ils sont individuellement transférés dans les puits de culture d'une plaque de culture "24 trous". Chaque clone est ensuite progressivement amplifié, en présence de généticine ou de phléomycine, d'abord dans les puits d'une plaque de culture "12 trous", puis "6 trous" pour être ensuite amplifié en boites de culture cellulaire.

### G- Construction d'un adénovirus recombinant portant une délétion dans les gènes E1- IVa2-E4.

### G.1 - Construction d'un adénovirus ΔE1-ΔIVa2-ΔE4 par cotransfection d'un virus helper portant le gène IVa2.

Par exemple, l'adénovirus ΔE1-ΔIVa2-ΔE4 a pu être préparé selon les deux protocoles suivants:
(a) l'ADN d'un virus ΔE1-ΔE4 digéré par Cla1 (par exemple AdRSVβgal-dl1004, AdRSVβgal-dl1007 ou AdRSVβgal-dl1014 décrit dans "Efficient dual transcomplementation of adenovirus E1 and E4 regions from a 293-derived cell line expressing a minimal E4 functional unit" de P. YEH et al., J. Virology, sous-presse), l'ADN du plasmide pCO2 digéré par Xmn1 et l'ADN du virus helper pAC2 ont été cotransfectés dans des cellules 293 E4 en présence de phosphate de calcium.
(b) l'ADN d'un virus ΔE1-ΔE4 digéré par Clal, l'ADN du plasmide pC06 digéré par Xmn1 et l'ADN du virus helper pAC2 ont été cotransfectés dans des cellules 293 E4 en présence de phosphate de calcium.

### G.2- Construction d'un adénovirus ΔE1-ΔIVa2-ΔE4 dans des lignées cellulaires transcomplémentant les fonctions IVa2 et E4.

Dans les clones cellulaires exprimant les régions IVa2 et E4 de l'adénovirus Ad5 et résistants à la phléomycine, l'adénovirus ΔE1-ΔIVa2-ΔE4 a pu être préparé selon les deux protocoles suivants:
(a) l'ADN d'un virus ΔE1-ΔE4 digéré par Cla1 et l'ADN du plasmide pCO2 digéré par Xmn1 ont été cotransfectés dans les cellules en présence de phosphate de calcium.
(b) l'ADN d'un virus ΔE1 -ΔE4 digéré par Clal et l'ADN du plasmide pC06 digéré par Xmn1 ont été cotransfectés dans les cellules en présence de phosphate de calcium.

## Revendications

1. Adénovirus recombinant défectif **caractérisé en ce qu'**il comprend les ITR et une région d'encapsidation et **en ce que** le gène IVa2 au moins est inactivé.

2. Adénovirus recombinant selon la revendication 1 **caractérisé en ce que** le gène IVa2 est inactivé par mutation et/ou délétion d'une ou de plusieurs bases.

3. Adénovirus recombinant selon la revendication 2 **caractérisé en ce que** le gène IVa2 est inactivé par délétion partielle.

4. Adénovirus recombinant selon la revendication 3 **caractérisé en ce que** le gène IVa2 est inactivé par délétion d'un fragment allant du nucléotide 4106 au nucléotide 5186.

5. Adénovirus recombinant selon l'une quelconque des revendications précédentes **caractérisé en ce qu'**il comporte une séquence d'acide nucléique hétérologue.

6. Adénovirus recombinant défectif **caractérisé en ce qu'**il comprend les ITR et une région d'encapsidation et **en ce qu'**il porte une délétion de tout ou partie des gènes E1 et IVa2.

7. Adénovirus recombinant défectif **caractérisé en ce qu'**il comprend les ITR et une région d'encapsidation et **en ce qu'**il porte une délétion au niveau du gène E1, allant du nucléotide 382 au nucléotide 3446, et une délétion au niveau du gène IVa2, allant du nucléotide 4106 au nucléotide 5186.

8. Adénovirus recombinant défectif **caractérisé en ce qu'**il comprend les ITR et une région d'encapsidation et **en ce qu'**il porte une délétion de tout ou partie des gènes E4 et IVa2.

9. Adénovirus recombinant défectif **caractérisé en ce qu'**il comprend les ITR et une région d'encapsidation et **en ce qu'**il porte une délétion de tout ou partie des gènes E1, IVa2 et E3.

10. Adénovirus recombinant défectif **caractérisé en ce qu'**il comprend les ITR et une région d'encapsidation et **en ce qu'**il porte une délétion de tout ou partie des gènes E1, IVa2 et E4.

11. Adénovirus recombinant défectif **caractérisé en ce qu'**il comprend les ITR et une région d'encapsidation et **en ce qu'**il porte une délétion de tout ou partie des gènes E1, IVa2, E3 et E4.

12. Adénovirus recombinant défectif **caractérisé en ce qu'**il comprend les ITR et une région d'encapsidation et **en ce qu'**il porte une délétion de tout ou partie des gènes E1, IVa2, E3, L5 et E4.

13. Adénovirus selon l'une des revendications précédentes **caractérisé en ce qu'**il est d'origine humaine, animale, ou mixte.

14. Adénovirus selon la revendication 13 **caractérisé en ce que** les adénovirus d'origine humaine sont choisis parmi ceux classés dans le groupe C, de préférence parmi les adénovirus de type 2 ou 5 (Ad 2 ou Ad 5).

15. Adénovirus selon la revendication 13 **caractérisé en ce que** les adénovirus d'origine animale sont choisis parmi les adénovirus d'origine canine, bovine, murine, ovine, porcine, aviaire et simienne.

16. Adénovirus selon l'une des revendications précédentes **caractérisé en ce qu'**il comprend en outre une partie du gène E3 codant pour la protéine gp19K sous contrôle d'un promoteur hétérologue.

17. Adénovirus selon la revendication 5 **caractérisé en ce que** la séquence d'acide nucléique hétérologue comporte un gène thérapeutique et/ou un gène codant pour des peptides antigéniques.

18. Adénovirus selon la revendication 5 **caractérisé en ce que** la séquence d'acide nucléique hétérologue comprend également une région promotrice de la transcription fonctionnelle dans la cellule, l'organe et/ou l'organisme infecté.

19. Adénovirus selon la revendication 5 **caractérisé en ce que** la séquence d'acide nucléique hétérologue comprend en outre une séquence de sécrétion.

20. Adénovirus recombinant défectif **caractérisé en ce qu'**il comprend les 1TR et une région d'encapsidation et **en ce qu'**il porte une délétion allant du nucléotide 382 au nucléotide 3446.

21. Composition pharmaceutique comprenant au moins un adénovirus recombinant défectif selon l'une des revendications 1 à 20.

22. Composition pharmaceutique selon la revendication 21 comprenant un véhicule pharmaceutiquement acceptable pour une formulation injectable.

## Claims

1. A defective recombinant adenovirus, **characterized in that** it includes the ITR genes and a region of encapsidation **in that** the IVa2 gene at least is inactivated.

2. The recombinant adenovirus according to claim 1, **characterized in that** the IVa2 gene is inactivated by mutation and/or deletion of one or more bases.

3. The recombinant adenovirus according to claim 2, **characterized in that** the IVa2 gene is inactivated by partial deletion.

4. The recombinant adenovirus according to claim 3, **characterized in that** the IVa2 gene is inactivated by partial deletion of a fragment going from nucleotide 4106 to nucleotide 5186.

5. The recombinant adenovirus according to one of the preceding claims, **characterized in that** it comprises a heterologic nucleic acid sequence.

6. The defective recombinant adenovirus, **characterized in that** it includes the ITR genes and an encapsidation region and **in that** it carries a deletion of all or part of the E1 and IVa2 genes.

7. The defective recombinant adenovirus, **characterized in that** it includes the ITR genes and an encapsidation region and **in that** it carries a deletion at the level of the E1 gene, going from the 382 nucleotide to the 3446 nucleotide, and a deletion at the level of the IVa2 gene, going from the 4106 nucleotide to the 5186 nucleotide.

8. The defective recombinant adenovirus, **characterized in that** it includes the ITR genes and an encapsidation region and **in that** it carries a deletion of all or part of the E4 and IVa2 genes.

9. The defective recombinant adenovirus **characterized in that** it includes the ITR genes and an encapsidation region and **in that** it carries a deletion of all or part of the E1, IVa2 and E3 genes.

10. The defective recombinant adenovirus, **characterized in that** it includes the ITR genes and an encapsidation region and **in that** it carries a deletion of all or part of the E1, IVa2 and E4 genes.

11. The defective recombinant adenovirus, **characterized in that** it includes the ITR genes and an encapsidation region and **in that** it carries a deletion of all or part of the E1, IVa2, E3 and E4 genes.

12. The defective recombinant adenovirus, **characterized in that** it includes the ITR genes and an encapsidation region and **in that** it carrtie a deletion of all or part of the E1, IVa2, E3, I5 and E4 genes.

13. The adenovirus according to one of the preceding claims, **characterized in that** it is of human, animal or mixed origin.

14. The adenovirus according to claim 13, **characterized in that** the adenoviruses of human origin are chosen among those classified in group C, preferably among the adenovirus of type 2 or 5 (Ad 2 or Ad 5).

15. The adenovirus according to claim 13, **characterized in that** the adenoviruses of human origin are chosen among the adenoviruses of canine, bovine, murine, ovine, porcine, aviary and simian.

16. The adenovirus according to one of the preceding claims, **characterized in that** it additionally includes a part of the E3 gene coding for the protein gpl9K under control of a heterologic promoter.

17. The adenovirus according to claim 5, **characterized in that** the heterologic nucleic acid sequence comprises a therapeutic gene and/or a gene coding for antigenic peptides.

18. The adenovirus according to claim 5, **characterized in that** the heterologic nucleic acid sequence likewise comprises a promoter region of the functional transcription in the cell, the organ and/or the infected organism.

19. The adenovirus according to claim 5, **characterized in that** the heterologic nucleic acid sequence additionally includes a secretion sequence.

20. The defective recombinant adenovirus, **characterized in that** it includes the ITR genes and an encapsidation region and **in that** it carries a deletion going from the 382 nucleotide to the 3446 nucleotide.

21. A pharmaceutical composition including at least one acceptable defective recombinant adenovirus according to one of claims 1 through 20.

22. The pharmaceutical composition according to claim 21 including a pharmaceutically acceptable vehicle for an injectable formulation.

## Patentansprüche

1. Defektiver rekombinanter Adenovirus, **dadurch gekennzeichnet, dass** er die ITRs und eine Verpackungsregion umfasst, und dass mindestens das Gen IVa2 inaktiviert ist.

2. Rekombinanter Adenovirus nach Anspruch 1, **dadurch gekennzeichnet, dass** das Gen IVa2 durch Mutation und/oder Deletion einer oder mehrerer Basen inaktiviert ist.

3. Rekombinanter Adenovirus nach Anspruch 2, **dadurch gekennzeichnet, dass** das Gen IVa2 durch partielle Deletion inaktiviert ist.

4. Rekombinanter Adenovirus nach Anspruch 3, **dadurch gekennzeichnet, dass** das Gen IVa2 durch Deletion eines Fragmentes inaktiviert ist, welches von Nukleotid 4106 bis Nukleotid 5186 reicht.

5. Rekombinanter Adenovirus nach einem der vorherigen Ansprüche, **dadurch gekennzeichnet, dass** er eine heterologe Nukleinsäuresequenz umfasst.

6. Defektiver rekombinanter Adenovirus, **dadurch gekennzeichnet, dass** er die ITRs und eine Verpackungsregion umfasst, und dass er eine Deletion der vollständigen Gene E1 und IVa2 oder von Teilen dieser trägt.

7. Defektiver rekombinanter Adenovirus, **dadurch gekennzeichnet, dass** er die ITRs und eine Verpackungsregion umfasst, und dass er eine Deletion im Bereich des Gens E1 trägt, welche von Nukleotid 382 bis Nukleotid 3446 reicht sowie eine Deletion im Bereich des Gens IVa2, welche von Nukleotid 4106 bis Nukleotid 5186 reicht.

8. Defektiver rekombinanter Adenovirus, **dadurch gekennzeichnet, dass** er die ITRs und eine Verpackungsregion umfasst, und dass er eine Deletion der vollständigen Gene E4 und IVa2 oder von Teilen dieser trägt.

9. Defektiver rekombinanter Adenovirus, **dadurch gekennzeichnet, dass** er die ITRs und eine Verpackungsregion umfasst, und dass er eine Deletion der vollständigen Gene E1, IVa2 und E3 oder von Teilen dieser trägt.

10. Defektiver rekombinanter Adenovirus, **dadurch gekennzeichnet, dass** er die ITRs und eine Verpackungsregion umfasst, und dass er eine Deletion der vollständigen Gene E1, IVa2 und E4 oder von Teilen dieser trägt.

11. Defektiver rekombinanter Adenovirus, **dadurch gekennzeichnet, dass** er die ITRs und eine Verpackungsregion umfasst, und dass er eine Deletion der vollständige Gene E1, IVa2, E3 und E4 oder von Teilen dieser trägt.

12. Defektiver rekombinanter Adenovirus, **dadurch gekennzeichnet, dass** er die ITRs und eine Verpackungsregion umfasst, und dass er eine Deletion der vollständigen Gene E1, IVa2, E3, L5 und E4 oder von Teilen dieser trägt.

13. Adenovirus nach einem der vorherigen Ansprüche, **dadurch gekennzeichnet, dass** er menschlichen, tierischen oder gemischten Ursprungs ist.

14. Adenovirus nach Anspruch 13, **dadurch gekennzeichnet, dass** die Adenoviren menschlichen Ursprungs ausgewählt sind aus denen, die in Gruppe C klassifiziert sind, vorzugsweise aus den Adenoviren des Typs 2 oder 5 (Ad2 oder Ad5).

15. Adenovirus nach Anspruch 13, **dadurch gekennzeichnet, dass** die Adenoviren tierischen Ursprungs ausgewählt sind aus Adenoviren von Hund, Rind, Maus, Schaf, Schwein, Vogel und Affe.

16. Adenovirus nach einem der vorherigen Ansprüche, **dadurch gekennzeichnet, dass** er ferner einen Teil des Gens E3 umfasst, welcher für das Protein gp19K kodiert, unter Kontrolle eines heterologen Promotors.

17. Adenovirus nach Anspruch 5, **dadurch gekennzeichnet, dass** die heterologe Nukleinsäuresequenz ein therapeutisches Gen und/oder ein Gen umfasst, welches für antigene Peptide kodiert.

18. Adenovirus nach Anspruch 5, **dadurch gekennzeichnet, dass** die heterologe Nukleinsäuresequenz ebenfalls eine transkriptionelle Promotorregion umfasst, welche in der infizierten Zelle, dem infizierten Organ und/oder Organismus funktionsfähig ist.

19. Adenovirus nach Anspruch 5, **dadurch gekennzeichnet, dass** die heterologe Nukleinsäuresequenz ferner eine sekretorische Sequenz umfasst.

20. Defektiver rekombinanter Adenovirus, **dadurch gekennzeichnet, dass** er die ITRs und eine Verpackungsregion umfasst, und dass er eine Deletion trägt, die von Nukleotid 382 bis Nukleotid 3446 reicht.

21. Pharmazeutische Zusammensetzung, die mindestens einen defektiven rekombinanten Adenovirus nach einem der Ansprüche 1 bis 20 umfasst.

22. Pharmazeutische Zusammensetzung nach Anspruch 21, die einen für eine injizierbare Formulierung geeigneten pharmazeutischen Träger umfasst.


### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96
   (ii) TITRE DE L' INVENTION: Vecteurs viraux et utilisation en thérapie génique
   (iii) NOMBRE DE SEQUENCES: 2
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
      CCTTAGCCCG GGCTAAGGCA TG 22
(2) INFORMATIONS POUR LA SEQ ID NO: 2:
   (i) CARACTERISTIQUES DE LA SEQUENCE:
      (A) LONGUEUR: 61 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
      TCGACGGATC CCTTAAGGTT GACGCCGCCA CCATGGAAAC CAGAGGGCGA AGACCGGCAG C 61